# EUROPEAN PATENT SPECIFICATION

(11) **EP 2 656 871 B1**
(45) Date of publication and mention of the grant of the patent: **09.12.2020**
(21) Application number: 13178193.2
(22) Date of filing: 23.06.2008
(51) Int. Cl.: A61M 25/10, A61F 2/97, A61F 2/958

(54) **MEDICAL DEVICE DELIVERY SYSTEM WITH SHEATH HAVING BALLOON-RELATIVE POSITION**
SYSTEM ZUR BEREITSTELLUNG EINER MEDIZINISCHEN VORRICHTUNG MIT UMMANTELUNG MIT BALLONRELATIVER POSITION
SYSTÈME DE DÉLIVRANCE DE DISPOSITIF MÉDICAL AVEC GAINE À POSITION RELATIVE DU BALLON

(30) Priority: 21.06.2007 US 766198; 30.10.2007 US 929958; 31.10.2007 US 930634
(43) Date of publication of application: 30.10.2013
(62) Divisional of application: 08771745.0
(73) Proprietor: Merit Medical Ireland Limited, Dublin 1 (IE)
(72) Inventor: Slattery, David, Kinvara (IE); Kelly, Damian, Loughrea, Co. Galway (IE); O'Connor, Therese, Athenry (IE); Kerins, Joseph, Turlough, Co. Mayo (IE); Gilmore, Michael, Loughrea, Co. Galway (IE); Steckel, Mark, Sharon, MA 02067 (US)
(74) Representative: LLR

(56) References cited:
- WO-A-01/97715
- WO-A1-2009/009636
- WO-A2-02/22053
- US-A1- 2005 125 021

## Description

### FIELD OF THE INVENTION

The present invention relates to a delivery system for deployment of a medical device, e.g., a self-expanding vascular device, in the vasculature of a patient. More particularly, a delivery system having a sheath with portions specifically positioned relative to a balloon positioned therein is described.

### BACKGROUND OF THE INVENTION

As is known, treatment of vascular blockages due to any one of a number of conditions, such as arteriosclerosis, often comprises balloon dilatation and treatment of the inner vessel wall by placement of a stent. These stents are positioned to prevent restenosis of the vessel walls after the dilatation. Other devices, often referred to as drug eluting stents, are now being used to deliver medicine to the vessel wall to also help reduce the occurrence of restenosis. These stents, i.e., tubular prostheses, typically fall into two general categories of construction. The first category of prosthesis is made from a material that is expandable upon application of a controlled force applied by, for example, a balloon portion of a dilatation catheter upon inflation. The expansion of the balloon causes the compressed prosthesis to expand to a larger diameter and then left in place within the vessel at the target site. The second category of prosthesis is a self-expanding prosthesis formed from, for example, shape memory metals or super-elastic nickel-titanium (NiTi or Nitinol) alloys, that will automatically expand from a compressed or restrained state when the prosthesis is advanced out of a delivery catheter and into the blood vessel.

Some known prosthesis delivery systems for implanting self-expanding stents include an inner lumen upon which the compressed or collapsed prosthesis is mounted and an outer restraining sheath that is initially placed over the compressed prosthesis prior to deployment. When the prosthesis is to be deployed in the body vessel, the outer sheath is moved in relation to the inner lumen to "uncover" the compressed prosthesis, allowing the prosthesis to move to its expanded condition. Some delivery systems utilize a "push-pull" type technique in which the outer sheath is retracted while the inner lumen is pushed forward. Still other systems use an actuating wire that is attached to the outer sheath. When the actuating wire is pulled to retract the outer sheath and deploy the prosthesis, the inner lumen must remain stationary, preventing the prosthesis from moving axially within the body vessel.

There have been, however, problems associated with these delivery systems. For example, systems that rely on a "push-pull design" can experience movement of the collapsed prosthesis within the body vessel when the inner lumen is pushed forward. This movement can lead to inaccurate positioning and, in some instances, possible perforation of the vessel wall by a protruding end of the prosthesis. Systems that utilize an actuating wire design will tend to move to follow the radius of curvature when placed in curved anatomy of the patient. As the wire is actuated, tension in the delivery system can cause the system to straighten. As the system straightens, the position of the prosthesis changes because the length of the catheter no longer conforms to the curvature of the anatomy. This change of the geometry of the system within the anatomy can also lead to inaccurate prosthesis positioning.

Other delivery systems are known where a self-expanding stent is kept in its compressed state by a sheath positioned about the prosthesis. A balloon portion of the delivery catheter is provided to rupture the sheath and, therefore, release the prosthesis.

As shown in U.S. Patent No. 6,656,213, the stent may be provided around the balloon, with the sheath around the stent, that is, the balloon, stent, and sheath are co-axially positioned, such that expansion of the balloon helps to expand the self-expanding stent as well as rupture the sheath. In other embodiments, the balloon is outside the stent and the sheath is around both the balloon and the stent.

To facilitate the rupturing of the sheath, it is further known to provide perforations in the sheath. The intention of the perforations is to make the rupturing or separation of the sheath easier upon expansion of the balloon. While the perforations may help to control the rupturing of the sheath by providing a "weak" portion, the dynamics of sheath rupturing are still not well controlled.

Alternative solutions are disclosed in US2002072789 and in US2005125021.

There is, therefore a need for a mechanism to reliably deliver a self-expanding stent, enclosed in a sheath, with repeatable and known operating characteristics.

Further, there have been issues, with the sheath having an adverse effect on the vessel as the delivery system is positioned. In some instances, the sheath has been "caught" on lesions that are found in the vessel.

There is, therefore, also a need for a sheath that does not interfere with positioning of the delivery system.

### SUMMARY OF THE INVENTION

A delivery system according to the invention comprises: a catheter having a distal end and a proximal end; a balloon portion positioned at the distal end of the catheter; a medical device, having a compressed state and an expanded state, positioned about the balloon portion; and a sheath positioned about the medical device to hold the medical device in the compressed state. The sheath comprises: a distal sheath portion located at a distal end of the sheath, the distal sheath portion having a first diameter and a first longitudinal length; a transition portion, of a second longitudinal length, having a distal end located adjacent the proximal end of the distal sheath portion, the distal end of the transition portion being of the first diameter and having a proximal end with a second diameter greater than the first diameter; a body portion of a third longitudinal length, having a distal end adjacent a proximal end of the transition portion, the body portion being of the second diameter; an opening provided in an outer surface of the sheath; and, the sheath comprises material with grain oriented along a longitudinal axis of the sheath wherein the opening is an initiation slit of a predetermined length oriented substantially in parallel with the material grain.

### BRIEF DESCRIPTION OF THE DRAWINGS

The above and further advantages of the present invention may be better understood by referring to the following description in conjunction with the accompanying drawings in which:
Figure 1A is a view of a device delivery system;
Figure 1B is a cross-sectional view of the device delivery system along line 1 B-1 Bas shown in Fig. 1A;
Figures 2A and 28 are views of a portion of a device delivery system;
Figure 3 is a cross-sectional view of a dual-wing PTCA balloon;
Figure 4 is a cross-sectional view of the dual-wing PTCA balloon of Fig. 3 in a bi-folded configuration and wrapped within a sheath;
Figure 5 is a cross-sectional view of the partially expanded PTCA balloon of Figures 3 and 4.
Figure 6 is a perspective view of an initial cut positioned on a sheath;
Figure 7 is a cross-sectional view of a tri-wing PTCA balloon;
Figure 8 is a cross-sectional view of the tri-wing PTCA balloon of Fig. 7 in a tri-folded configuration and wrapped within a sheath;
Figure 9 is a cross-sectional view of a dual-wing PTCA balloon in a U-fold configuration and wrapped within a sheath;
Figure 10 is a method of placing a sheath split with respect to an orientation of a balloon placed within;
Figure 11 is an alternate method of loading a device on a delivery system and orienting the splits in the sheath with a balloon;
Figure 12 is a perspective view of the dual-wing PTCA balloon of Fig. 3;
Figure 13 is a representation of a sheath similar to the one shown in Figure 7 while being positioned in a vessel;
Figure 14 is a profiled sheath in accordance with one embodiment of the present invention;
Figure 15 is a representation of the sheath shown in

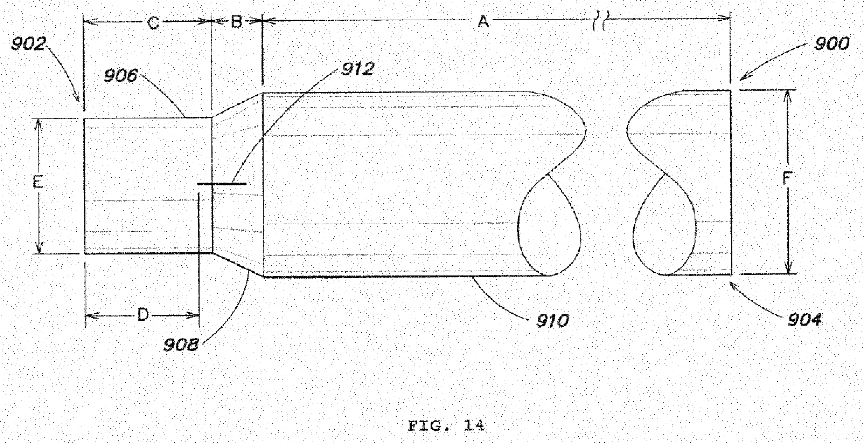

Figure 14 positioned on a delivery system;

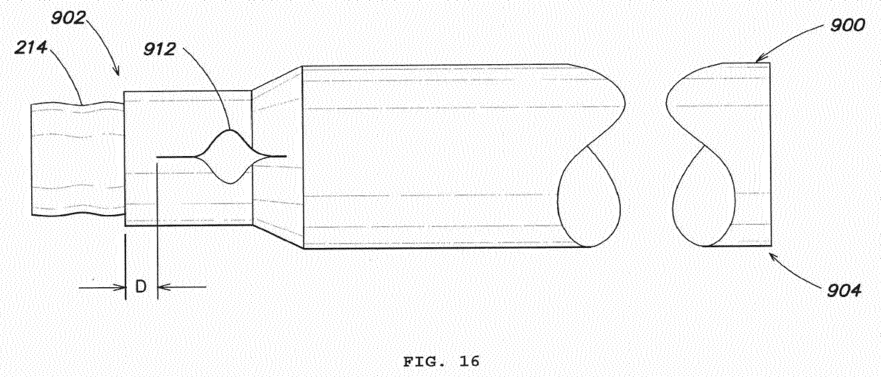

Figure 16 is a representation of a portion of the sheath of Figure 14 as the sheath is being expanded:

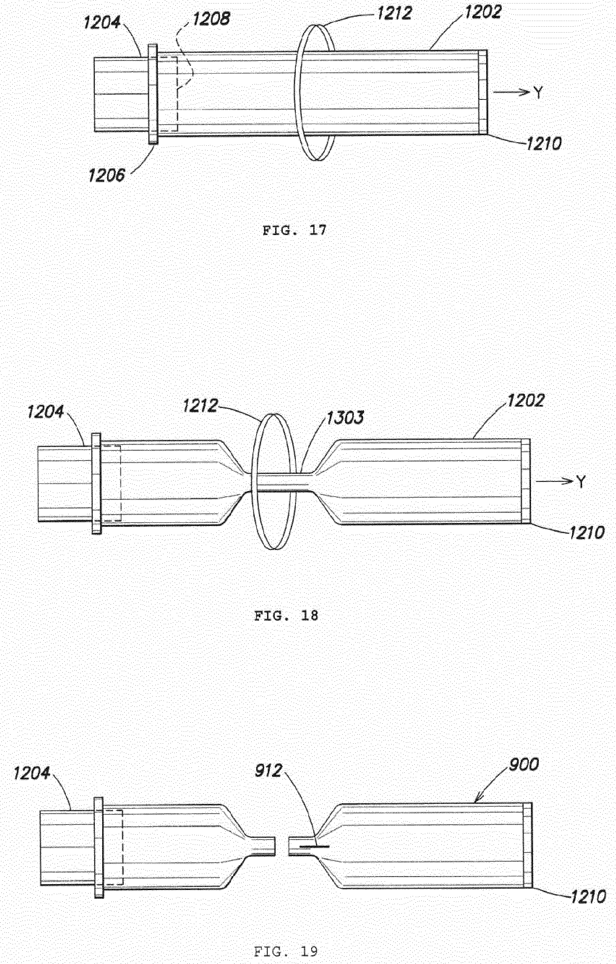

Figures 17 - 19 represent one embodiment of a method of manufacturing the sheath of Figure 14; and

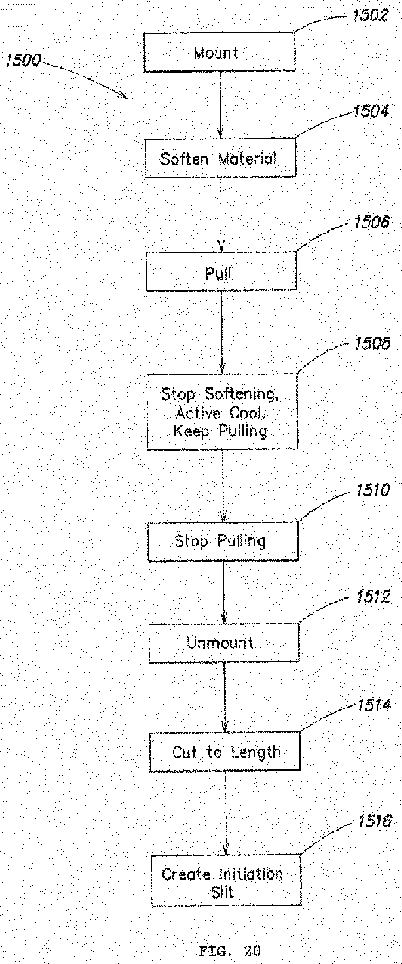

Figure 20 is a flowchart of the steps of an embodiment of a method of manufacturing the sheath of Figure 14.

### DETAILED DESCRIPTION

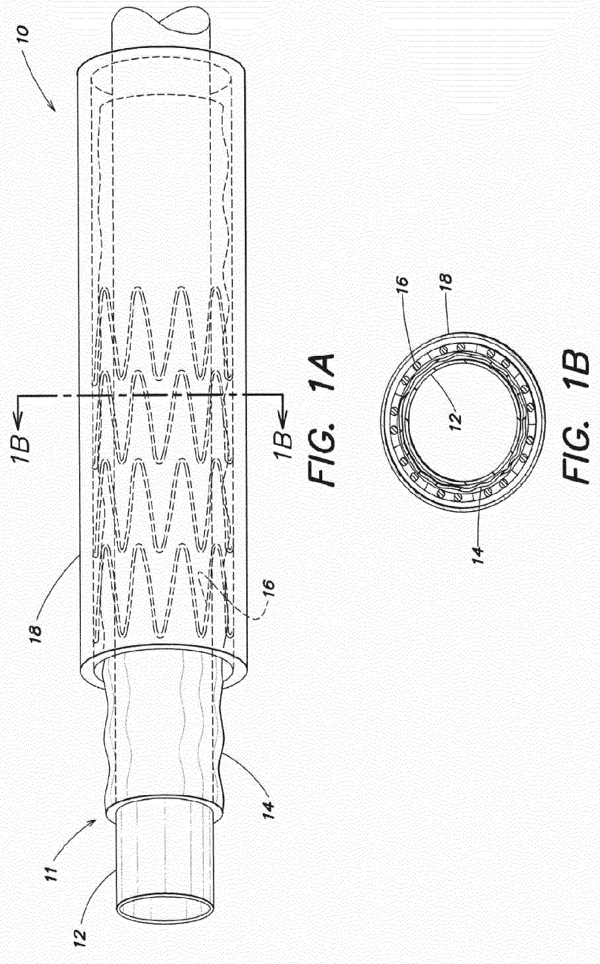

A medical device delivery system, as shown in Fig. 1A, includes a delivery catheter 12 with a balloon 14 positioned at, or enclosing, a distal end 11 of the catheter 12. As is known, a lumen is provided to inflate the balloon 14 as necessary during the procedure to deliver a device 16, for example, a stent that is placed at the distal end of the catheter 12 and around the balloon 14. As per the present discussion, the device 16 is a self-expanding device and, therefore, a sheath 18 is also disposed at the distal end 1 1 of the catheter 12 so as to enclose the device 16 and the balloon 14. The sheath 18 is attached to the catheter 12 at some point proximal to the distal end 1 1 of the catheter 12.

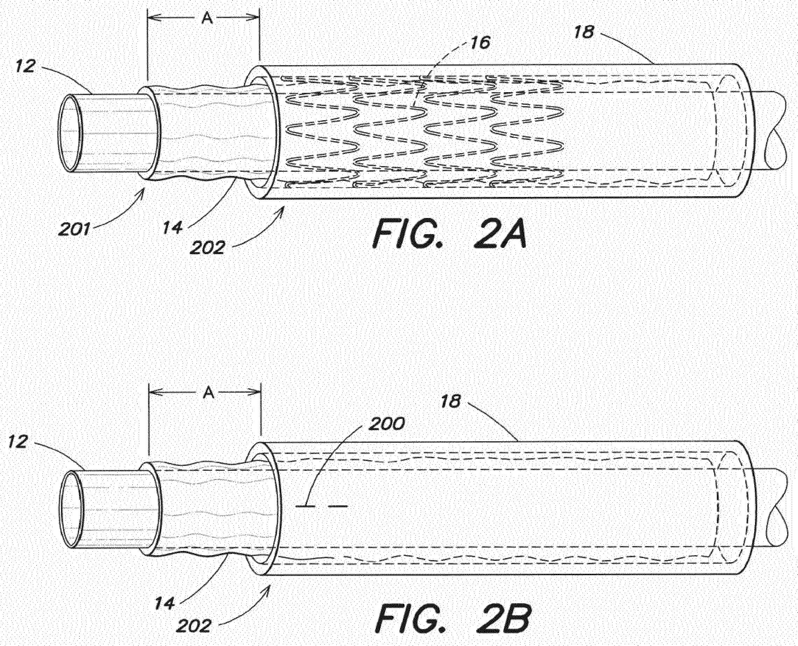

A cross section of the system 10, along line 1 B-1 B, is presented in Fig. 1B. As shown, the sheath 18 surrounds the stentor device 16 and the balloon 14 positioned on the catheter 12. Referring to Fig. 2A, a simpler representation of the system 10 of Fig. 1A, is shown where a distal end 202 of the sheath 18 is positioned a distance A proximally from a distal end 201 of the balloon 14. Placing the distal edge 202 of the sheath 18 a predetermined distance proximal to the distal end 201 of the balloon 14 allows for maximum effectiveness of the balloon 14 with respect to rupturing of the sheath 18. Referring now to Fig. 28, corresponding to Fig. 2A, with the device 16 removed for clarity, a perforation 200 is provided in the sheath 18. The perforation 200 is shown here at the distal end 202 of the sheath 18. The perforation 200 facilitates the separation or rupture of the sheath 18 as the balloon 14 is expanded. The perforation 200 may comprise, in one example, one or more discontinuous slits in the sheath material 18. Here, a slit does not involve the removal of sheath material.

Alternatively, the sheath 18 may be created by weakening a portion of the sheath 18 by chemical and/or mechanical means. Still further, the perforation 200 may comprise one or more holes, where each hole is created by the removal of sheath material. While the perforation 200 is shown here at the distal end of the sheath 18, of course, one of ordinary skill in the art would understand that were the sheath 18 to be connected to the catheter 12 at the distal end of the sheath 18, then the perforation 200 may be positioned at a proximal end of the sheath 18. Further, more than one perforation 200 may be provided, for example, one at each of the proximal and distal ends of the sheath 18, respectively.

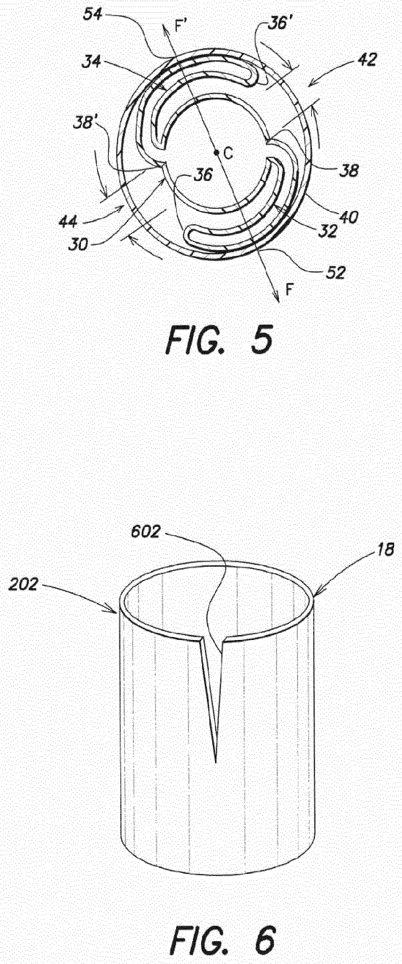

As an alternative to the perforation 200, a single initial cut 602, as shown in Fig. 6, may be implemented.

The sheath is made from a material having a grain, or fibers that can be longitudinally oriented, for example, PTFE. In general, the sheath 18, upon expansion of the balloon 14, will tear along the perforation 200 or initial cut 602 in substantially a straight line following a longitudinal axis of the sheath 18 as defined, generally, by the catheter 12.

The expansion of the balloon causes the sheath to rupture. Once the sheath ruptures, the stent expands and is released into the vessel. The balloon pressure that will cause a sheath 18 to split, however, is not consistent in previously known systems. Tests have shown that the sheaths from a batch of balloon-mounted systems will not always split at the same balloon pressure.

Experiments were conducted by the present inventors, using an external polymer sheath 18, made from PTFE, and a PTCA balloon catheter. In the test setup, the PTCA balloon catheter included a 2.0 mm by 30 mm nylon balloon 14. The particular type of balloon 14 that was used in these experiments exhibited semi-compliant behavior, in that it has a compliance of approximately 5% at 2 mm with 6 atmospheres of nominal pressure, i.e., the balloon diameter ranges from 1.9 mm to 2.1 mm at 6 atmospheres. The external polymer sheath 18 was provided in two sizes: a) 1,0922 mm (0,043 inches) inner diameter by 0,0508 mm (0,002 inches) wall thickness; and b) 1,1938 mm (0,047 inches) inner diameter by 0,0508 mm (0,002 inches) wall thickness. The sheath was positioned substantially as shown in Figs. 1A and 1B. Further, an initial cut was placed at the distal end of the sheath 18.

Five samples of each of sheath type a) and sheath type b) were externally loaded onto the 2.0 mm by 30 mm PTCA balloon catheter. The balloon was inflated in one, atmosphere intervals to a pressure until the polymer sheath fully split, i.e., the sheath split along the full length of the 30 mm PTCA balloon. As shown in Table 1 below, for sheath! type (a) the balloon pressure needed to fully split the sheath ranged from 4 to 8 atmospheres. The sheath of type (b) exhibited a full split with balloon pressures that ranged from 7 to 18 atmospheres.

**Table 1**

| **No.** | **Sheath** | **Split (atm) m)** | | **No.** | **Sheath** | **Split (atm)** |
|---|---|---|---|---|---|---|
| 1 | (a) | 4 | | 1 | (b) | 9 |
| 2 | (a) | 4 | | 2 | (b) | 9 |
| 3 | (a) | 8 | | | (b) | 12 |
| 4 | (a) | 6 | | 4 | (b) | 7 |
| 5 | (a) | 6 | | 5 | (b) | 18 |

This inconsistency in the balloon pressure required to fully split the polymer sheath appears to hinder the effectiveness of the sheath for delivery of a device. The wide range of balloon pressure values required to fully split the sheath renders a construction substantially as represented in Fig. 1A too variable to validate and subsequently too variable to use in everyday procedures.

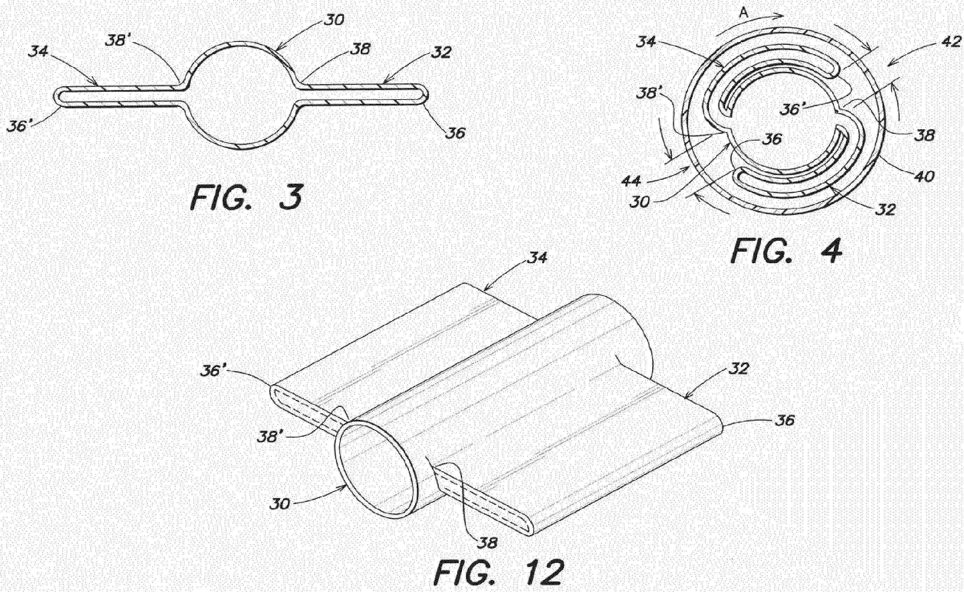

The present inventors recognized that the bi-folded wings of a PTCA catheter balloon could be used to aid in better controlling the splitting dynamics of the sheath. A deflated PTCA catheter balloon 30, shown in a perspective view in Figure 12 and in cross section in Fig. 3, includes, when the PTCA balloon 30 is vacuumed, two substantially equal wings 32, 34. Each wing has a wing tip 36 and a wing base 38. It should be noted that the catheter 12 and stent 16 are not shown in Figs. 3 and 4 for clarity although one of ordinary skill in the art would certainly understand the positioning of these components in a system according to the present disclosure.

Referring to Fig. 4, the PTCA balloon 30, once mounted on the delivery system, is folded such that the wings 32, 34 "wraparound" the body of the balloon 30 in such a way so as to not interfere with each other as the balloon 30 is inflated, i.e., a "wrap bi-fold" orientation. In general, a wing tip portion 36' of the wing 34 is folded along a circumferential direction A (shown by arrow) toward the base portion 38 of the wing 32. Similarly, the wing tip portion 36 of the wing 32 is folded toward the wing base portion 38' of the wing 34, continuing in the direction A. Looking along the axis of the system, as shown in Figure 4, the results of the folds of the balloon in this fashion are similar to a child's pinwheel. A sheath 40 is then provided over the folded balloon, and the device 16 (not shown) to keep the device 16 in a compressed state.

The present inventors have observed that placement of the perforation 200 or split 602 to take advantage of the mechanical leverage provided from the folded wings 32, 34 of the balloon 30 will aid in establishing a consistent and repeatable splitting of the sheath at a specific pressure, or relatively narrow range of pressures, of the balloon. In known systems, the split or perforation on the sheath were randomly placed, irrespective of any geometry of the balloon around which the sheath was disposed.

As found by the inventors of the present invention, there is an optimum area or areas on the circumference of the sheath at which to place the perforation 200 (running longitudinally) or initial cut 602. These locations around the circumference are determined by the folded balloon.

Referring to Fig. 4 a sheath 40 has been provided around a dual-wing balloon 30 in a wrap bi-folded configuration. Two placement areas 42, 44 along the circumference of the sheath 40 are defined. Placing the perforation 200 or cut 602 within at least one of these placement areas optimizes the tearing or rupturing of the sheath 40. These two areas 42, 44 are defined or predetermined with respect to the orientation of the folded balloon.

When the perforation 200 or initial cut 602 is placed anywhere within one of the areas 42, 44, the sheath 40 will split at a uniform and consistent and repeatable pressure of the balloon. It should be noted that one initial cut or perforation in either of the areas 42, 44 is sufficient to initiate the full split of the sheath 40. It has been observed, however, that a split or perforation may be placed in each of the areas 42, 44 to facilitate separation of the sheath 40.

A second set of experiments was performed where sheaths, with the same construction as those previously described, are provided around the PTCA balloon except that the perforation or initial cuts are placed in one of the areas 42, 44, i.e., relative to the orientation of the balloon 30. Once again, the balloon is inflated in increments of 1 atmosphere. As shown in Table 2, the balloon pressure necessary to fully split the sheath 40 was repeatedly 5 atmospheres.

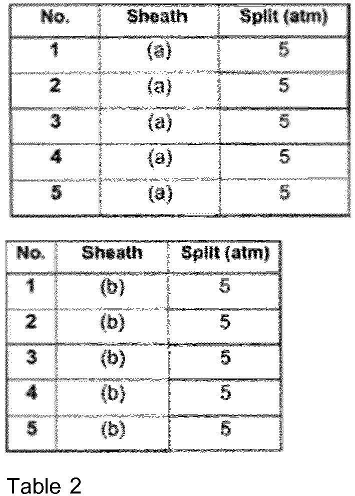

The specific placement of the initial cut 602 or perforation 200 with respect to the folded geometry or orientation of the balloon provides consistent and repeatable sheath splitting performance. The repeatability and consistency of obtaining a full split provides an advantage with respect to using a delivery system with a balloon expandable sheath to deliver a self-expanding medical device.

Thus, the present inventors have recognized that the folds or wings 32, 34 of the PTCA balloon 30 play a role in splitting the sheath 40, due to the placement of the split 602 or perforation 200. Further, optimum positions about the circumference of the sheath can be predetermined as a function of the balloon's placement and folded geometry about the catheter.

Referring to Fig. 5, the placement areas 42, 44 can be defined as those locations around the circumference of the sheath 40 at which the resultant force exerted by the wings 32, 34, against the sheath as the balloon is inflated, is at a maximum. It can be considered that the balloon 30 expands symmetrically from its center C as it is being inflated. The wings 32, 34 exert, respectively, forces F and F', against the sheath 40 at points 52, 54, respectively. The cumulative effect of the forces of the wings 32, 34 against the sheath 40 is maximized in the two placement areas 42, 44. Thus, placing a perforation or an initial cut in either or both of the placement areas 42, 44 provides for a repeatable and consistent splitting of the sheath 40 at a known pressure.

The placement areas 42, 44 located about the circumference of the sheath 40 may be considered to be defined as located generally halfway between circumferentially adjacent points where the balloon wings 32, 34 exert a respective force against the sheath 40 upon inflation of the balloon. The placement areas 42, 44, in one example are located along the circumference of the sheath within a portion of the circumference that is in a range of 40-60% of the distance between the points 52, 54.

Alternatively, the location of the placement areas 42, 44 may be described as being located between a wing tip 36 and a wing base 38 of adjacent wings of the balloon. As shown in Figure 5, due to the bi-fold of the balloon 30, the wing tip portion 36' is adjacent the wing base portion 38. The placement area 42 is, therefore, located substantially half-way between these two wing portions.

Advantageously, the placement areas 42, 44 are easily discernible by viewing the folded balloon within the sheath.

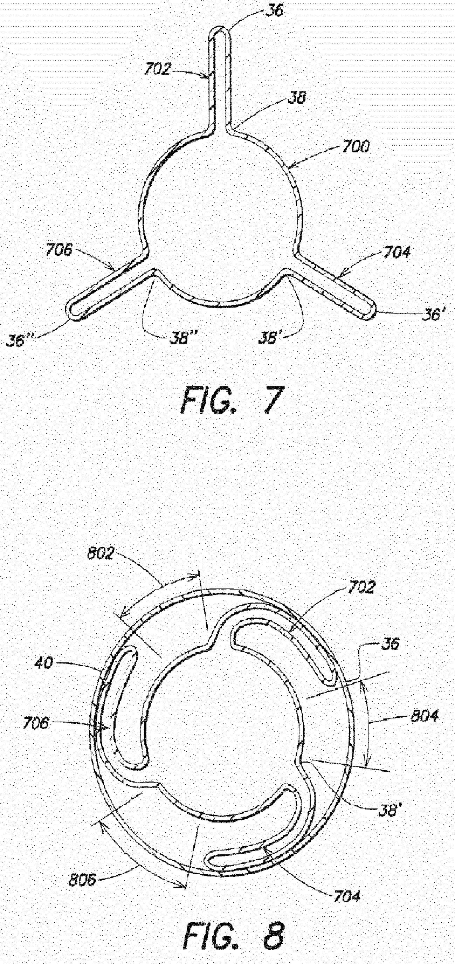

The balloon 30, as shown in Figure 3, is of a dual-wing design. Alternatively, a balloon 700 of a tri-wing design, as shown in cross-section in Figure 7, may be used. As shown, the balloon 700 has three wings 702, 704, 706 symmetrically disposed about the circumference of the balloon. Each of the wings has a wing tip 36 and a wing base 38.

When folded, and placed within a sheath 40, as shown in cross-section in Figure 8, placement areas 802, 804, 806 are positioned about the circumference of the sheath 40. Similar to the foregoing description, the placement areas 804, 806 are, respectively, located between adjacent wing tip portions 36 and wing base portions 38.

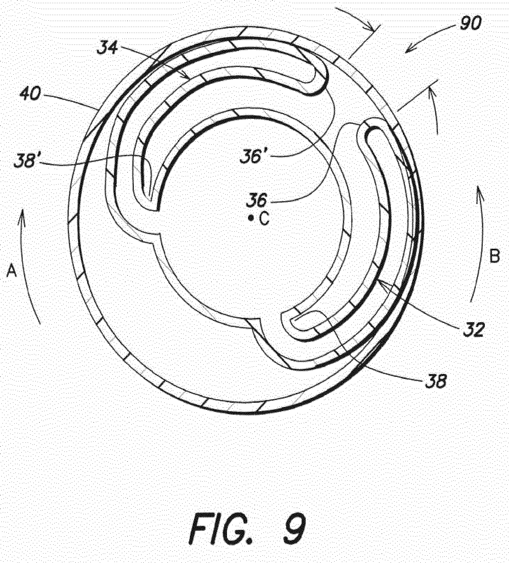

In yet another example, as shown in Figure 9, the dual-wing balloon is folded in a U-fold, where the wings 32, 34 have their respective wingtip portions 36, 36' adjacent one another. In this configuration, the wing 34 is wrapped in the circumferential direction A (as shown by the arrow A) while the wing 32 is wrapped in an opposite circumferential direction B (as shown by the arrow B) opposite that of direction A. The placement area 90 is then located along the circumference of the sheath 40 substantially midway between the wingtip portions 36, 36'. It is expected that as the balloon is inflated in this orientation the cumulative effect of the wing portions pushing on this sheath will be maximized within the placement area 90.

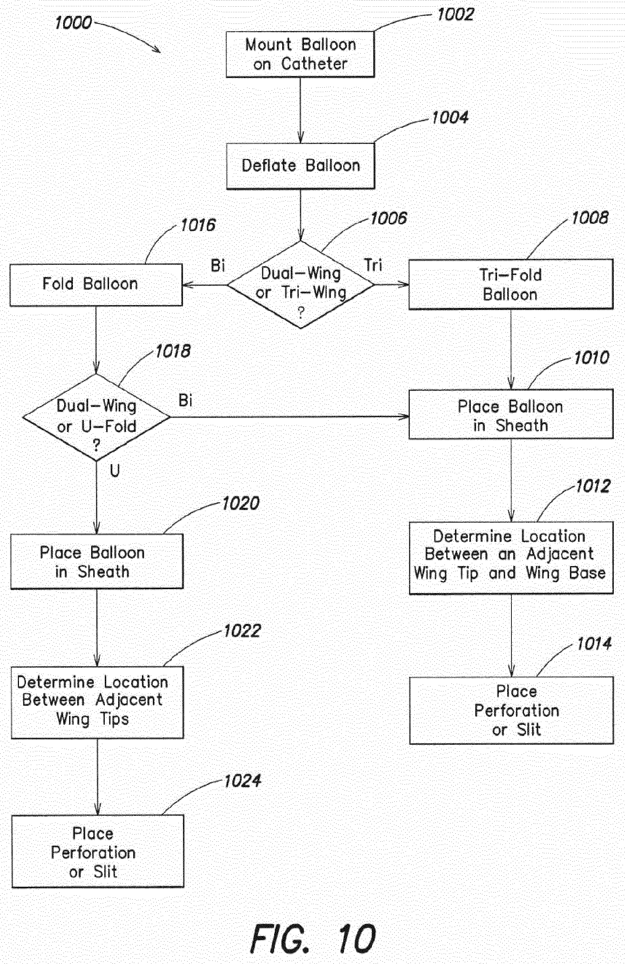

A method 1000 for assembling a delivery system as described above is shown, generally, in Figure 10. Initially, step 1002, the balloon is mounted on the catheter. For the sake of simplicity, reference to a medical device being mounted is not included in this description, however, one of ordinary skill in the art will understand where the medical device would be installed. Subsequently, step 1004, the balloon is mostly deflated, i.e., a vacuum is created within the balloon lumen. At step 1006 it has to be determined whether or not the balloon is of a dual-wing or tri-wing construction. If it is the latter, control passes to step 1008 where the balloon is folded in a tri-fold configuration. The balloon is then placed in the inside diameter of the device 16 and within the sheath, step 1010. One or more locations between an adjacent wing-tip and wing-base are then determined at step 1012. Once the location of the placement area is determined in step 1012, the perforation or slit is provided at step 1014. Returning to step 1006, if the balloon is of a dual-wing construction then control passes to step 1016 where the balloon is folded. At step 1018 it is determined as to whether or not the balloon was folded in a bi-fold configuration or aU-fold configuration. If it is determined that is the former configuration then control passes to step 1010 and operation continues as described above. If, however, it is the U-fold configuration then, at step 1020, the balloon is then placed in the inside diameter of the device 16 and within the sheath. Subsequently, step 1022, the location between adjacent wing tips about the circumference of the sheath is determined. Finally, step 1024, the perforation or slit is placed in the determined location.

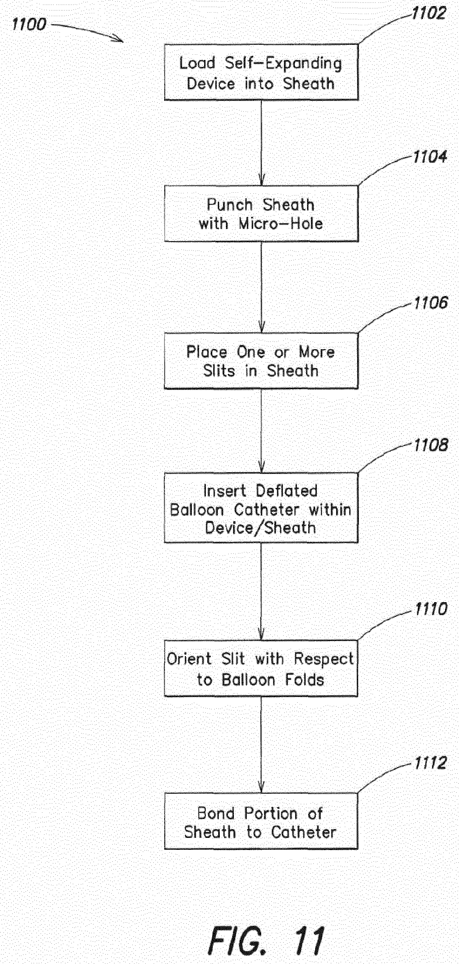

An alternate method 1 100 for assembling a system in accordance with another example of the present disclosure will now be described with respect to the flowchart shown in Figure 11. Initially, a self-expanding device is loaded into a sheath, step 1102. A micro-hole is then punched into the sheath, step 1 104, in order to facilitate the flow-through of liquid, for example, blood, as may be found in a vessel in which the device will be placed. One or more slits or perforations or holes are placed in the sheath, step 1106. A deflated balloon, with its wings folded in one of the orientations described above, is positioned on a catheter which is then inserted within the device/sheath assembly, step 1 108. The previously provided slit or perforation is then oriented with respect to the balloon fold, in accordance with the previously described process, step 1110. Once aligned, a portion of the sheath is bonded to the catheter to maintain this orientation, step 1 112.

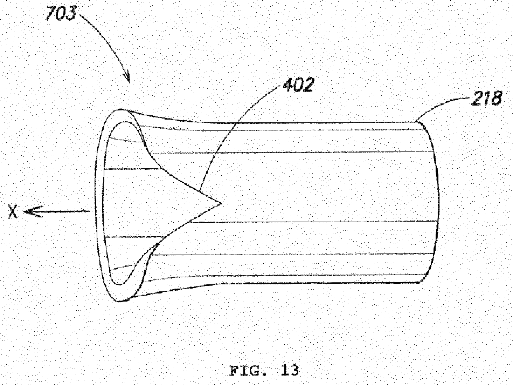

Turning now to Figure 13, as the delivery system, not shown, is maneuvered through the vessel in a distal direction X, as shown by the arrow, there have been incidents where a distal end 703 of a sheath 218 begins to spread apart and enlarge. One theory is that as the delivery system is inserted through a curved vasculature, portions of the sheath 218 extend in a direction opposite to that of the curving delivery system. It has been noted that this expansion of the sheath 218 is similar to an open mouth of a fish. This "fish-mouthing" of the sheath 218 is problematic as the sheath 218 may catch on lesions in the vessel and/or prevent the delivery system from properly tracking distally across a lesion. This interference with the tracking, or the catching on lesions, can lead to significant complications in the procedure, and may increase the time of the procedure, any of which can adversely affect patient safety.

The inventors of the present application have noted that adjustment of either the inner diameter of the sheath or the wall thickness of the material from which the sheath is made, does not sufficiently reduce the occurrence of fish-mouth. It appears that the initiation slit 402 is one of the leading factors that contributes to the size of the fish-mouth. In one series of experiments, the length of the initiation slit 402 was reduced from 1.5mm to .5mm and the amount of fish-mouth width, i.e., diameter, was substantially reduced. While the amount of fish-mouthing was reduced, however, the benefit of a lower and consistent pressure of the balloon portion necessary to consistently open, i.e., rupture, the sheath 218, was negatively affected. Thus, merely reducing the length of the initiation slit 402, while it does reduce the width of the fish-mouth, prevents consistent release of the device at a lower balloon pressure.

A profiled sheath 900, as shown in Figure 14 and representing the present invention, reduces lesion crossing issues and, therefore, reduces the occurrences of complications that may adversely affect the proper and safe delivery of a self-expanding medical device. The profiled sheath 900 includes a distal end 902 and a proximal end 904. A distal sheath portion 906 is located at the distal end 902 and has a corresponding longitudinal length C. Located proximal to the distal sheath portion 906 is a cone/transition portion 908 having a corresponding longitudinal length of B. Located proximal to the cone/transition portion 908 is a body portion 910 having a corresponding longitudinal length A. As shown, the distal sheath portion 906 has a corresponding width E and the body portion 910 has a corresponding width F. The cone/transition portion 908 transitions from the width E to the width F as between the distal sheath portion 906 and the body portion 910, respectively.

In the present description, reference to "width" is referring to the diameter of the tubular sheath. Further, while there is reference to "portions," e.g., distal sheath portion 906, the profiled sheath 900 is, in one embodiment, of a unitary construction.

The sheath 900 is made from material similar to that referenced above. Further, the grain direction of this material is oriented in a longitudinal direction along the profiled sheath 900 running from the distal end 902 to the proximal end 904.

An initiation opening 912 is provided across a junction or boundary between the distal sheath portion 906 and the cone/transition portion 908. A distal-most part of the opening 912 is located a distance D from the distal end 902 of the sheath 900. Thus, the opening 912 is "set back" from the distal end 902 of the sheath 900. It is advantageous to position the opening 912 across the boundary between the distal sheath portion 906 and the cone/transition portion 908. The opening 912 need not, however, be symmetrically positioned across the boundary.

The opening 912 may be implemented as a slice in the sheath material, where no material has been removed, and where there are sharp edges at each end of the opening 912. The sharp edges assist in the consistent splitting of the sheath. The opening 912 may be created by a slicing operation or a punching operation. The opening 912 may be implemented by operation of a sharp blade or a slicing laser device could be used. Alternatively, the opening 912 may result from an operation where material is removed, i.e., "punched out."

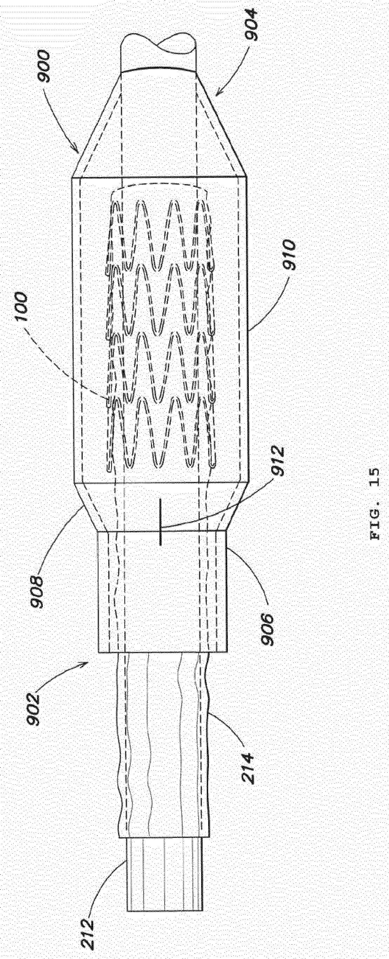

Referring now to Figure 15, the profiled sheath 900 is disposed about a self-expanding stent 100 and a balloon portion 214 of a delivery system 212 in substantially the same way as has been described above. As shown, the distal end 902 is located about a balloon portion 214, i.e., proximal to a distal end of the balloon portion 214. The sheath 900 is positioned with respect to the stent 100 such that the distal sheath portion 906 and the cone/transition portion 908 are located distal to a distal-most end of the device 100. In other words, the device 100 corresponds substantially with the body portion 910 of the profiled sheath 900. Similar to the description above, the proximal end 904 of the profiled sheath 900 is attached to the delivery catheter 212 to facilitate withdrawal of the ruptured sheath subsequent to deployment of the medical device 100.

In operation, as the balloon portion 214 is inflated, the opening 912 will expand as shown in Figure 16. The fibers of the material from which the profiled sheath 900 is made are oriented longitudinally, therefore, as the balloon portion 214 inflates, the opening 912 will expand and the profiled sheath 900 will rupture. The distance D is chosen to minimize the amount of rupturing of the profiled sheath 900 at the opening 912 due to tensile forces. In one embodiment, an opening 912 approximately 1.5mm long is placed not more than about 0.5mm from the distal end 902 of the sheath 900.

A profiled sheath 900 is made from any suitable material for a sheath as has been described above. To make the profiled sheath, the material is initially provided as a cylindrical tube of material 1202 and is attached to one end of a mandrel 1204, as shown in Figure 17. The material tube 1202 may be attached to a cap portion consisting of a ring 1206 and an inset portion 1208. A coupling ring or tab 1210 is attached to the free end of the material tube 1202. An RF coil 1212 is then positioned about the material tube 1202.

The RF coil 1212 is activated while, at the same time, a pulling force is applied to the free end 1210 in a direction Y, as shown in Figures 17 and 18. The active RF coil 1212 raises the temperature of the sheath material making it soft and malleable. In one example, raising the temperature of the PTFE material to about 230°C is sufficient to soften the material without melting. The activation of the RF coil 1212, in conjunction with the pulling force on the material 1202, causes the tube material to, generally, create a lengthened portion 1302 of the tube having a smaller diameter than the cylindrical tube initially possessed.

Once the desired profile has been obtained, the narrowed portion of the tube is then cut and the opening 912 is created, as shown in Figure 19. When the tab portion 1210 is removed, the profiled sheath 900 remains.

As shown in the flowchart of Figure 20, a method 1500 for manufacturing the profiled sheath 900, with respect to Figures 17-19, in accordance with one example of the present disclosure, begins with mounting the cylindrical material on the mandrel 1204, step 1502. Subsequently, step 1504, the sheath material is softened, e.g., via the RF coil, while the sheath material is pulled, step 1506. In one example, an amount of pressure used to grip the sheath is 6 bar while power to the RF coil is on for approximately 3 seconds. The heating is stopped, i.e., the RF coil is turned off, and the sheath is actively air-cooled with ambient air for about 8 seconds in one example while still maintaining a pulling force on the material, step 1508. In one example, the pull rate is approximately 2.3 mm/sec. After a predetermined amount of time, the pulling is stopped, step 1510, and the material is removed from the mandrel, step 1512. In one example, a stretched length is approximately 5.5 mm. The profiled sheath 900 is then cut to length, step 1514, and the initiation opening is created, step 1516.

While an RF coil has been described for softening the sheath material, a heater, microwave device, steam device, or infrared (IR) laser could also be used. Choosing the apparatus or method for softening the sheath material is within the capabilities of one of ordinary skill in the art.

In another example of the present disclosure, the profiled sheath 900 can be oriented with respect to the orientation of the folded balloons as described above. The initiation opening 912 can be positioned about the circumference at a location where the force from the opening of the wings from the balloon is at a local maximum.

It is to be understood that the present invention is not limited in its application to the details of construction and the arrangement of the components set forth in the foregoing description or illustrated in the drawings. The invention is capable of other embodiments or of being practiced or carried out in various ways. Also, it is to be understood that the phraseology and terminology employed herein are for the purpose of description and should not be regarded as limiting.

It is further appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

The present invention can be implemented with any balloon having two or more wings where the initial cut or perforation are placed in the sheath with respect to those points on the sheath at which the wings of the balloon exert force against the sheath as the balloon is being inflated.

Thus, in accordance with the teachings of the present invention, the placement of one or more initial cuts or series of perforations in a sheath that is provided to constrain a self-expanding device, for example, a stent prior to delivery, is determined with respect to a geometry and orientation of a folded balloon around which the sheath is provided.

Although various exemplary embodiments of the present invention have been disclosed, it will be apparent to those skilled in the art that changes and modifications can be made that will achieve some of the advantages of the invention without departing from the scope of the invention. It will be apparent to those reasonably skilled in the art that other components performing the same functions may be suitably substituted.

## Claims

1. A delivery system (212), comprising:
a catheter having a distal end (902) and a proximal end (904);
a balloon portion (214) positioned at the distal end of the catheter (12);
a medical device (100), having a compressed state and an expanded state, positioned about the balloon portion (214); and
a sheath (900) positioned about the medical device to hold the medical device (16, 100) in the compressed state,
the sheath (900) comprises:
a distal sheath portion (906) located at a distal end (902) of the sheath (900), the distal sheath portion (906) having a first diameter and a first longitudinal length;
a transition portion (908), of a second longitudinal length, having a distal end located adjacent the proximal end of the distal sheath portion, the distal end of the transition portion (908) being of the first diameter and having a proximal end with a second diameter greater than the first diameter;
a body portion (910) of a third longitudinal length, having a distal end adjacent a proximal end of the transition portion (908), the body portion (910) being of the second diameter;
an opening (912) provided in an outer surface of the sheath (900), a distal-most end of the opening (912) being located at a predetermined distance proximally from the distal end (902) of the sheath (900);
**characterized in that**
the sheath (900) comprises material with a grain oriented along a longitudinal axis of the sheath, and
wherein the opening (912) is an initiation slit of a predetermined length oriented substantially in parallel with the material grain.

2. The system of claim 1, **characterized in that** the opening (912) is provided so as to extend from the distal sheath portion to the transition portion (908).

3. The system of claim 1, **characterized in that**:
a distal-most end of the medical device (100) is proximal to a distal-most end of the sheath body portion.

4. The system of claim 1, **characterized in that** the opening (912) is approximately 1.5mm long and is placed not more than about 0.5mm from the distal end (902) of the sheath (900).

## Patentansprüche

1. Zufuhrsystem (212), umfassend:
einen Katheter mit einem distalen Ende (902) und einem proximalen Ende (904);
einen Ballonabschnitt (214), der an dem distalen Ende des Katheters (12) positioniert ist;
eine medizinische Vorrichtung (100), die einen komprimierten Zustand und einen expandierten Zustand aufweist und um den Ballonabschnitt (214) herum positioniert ist; und
eine Ummantelung (900), die um die medizinische Vorrichtung herum positioniert ist, um die medizinische Vorrichtung (16, 100) im komprimierten Zustand zu halten, wobei die Ummantelung (900) umfasst:
einen distalen Ummantelungsabschnitt (906), der sich an einem distalen Ende (902) der Ummantelung (900) befindet, wobei der distale Ummantelungsabschnitt (906) einen ersten Durchmesser und eine erste Länge in Längsrichtung aufweist;
einen Übergangsabschnitt (908) von einer zweiten longitudinalen Länge, der ein distales Ende aufweist, das neben dem proximalen Ende des distalen Ummantelungsabschnitts angeordnet ist, wobei das distale Ende des Übergangsabschnitts (908) den ersten Durchmesser aufweist und ein proximales Ende mit einem zweiten Durchmesser, der größer als der erste Durchmesser ist, aufweist;
einen Körperabschnitt (910) von einer dritten Länge in Längsrichtung, der ein distales Ende neben einem proximalen Ende des Übergangsabschnitts (908) aufweist, wobei der Körperabschnitt (910) den zweiten Durchmesser aufweist;
eine Öffnung (912), die in einer Außenfläche der Ummantelung (900) ausgebildet ist, wobei ein am weitesten distales Ende der Öffnung (912) in einer vorbestimmten Distanz proximal von dem distalen Ende (902) der Ummantelung (900) angeordnet ist;
**dadurch gekennzeichnet, dass**
die Ummantelung (900) Material mit einem Korn umfasst, das entlang einer Längsachse der Ummantelung orientiert ist, und
wobei die Öffnung (912) ein Initiierungsschlitz von einer zuvor festgelegten Länge ist, der im Wesentlichen parallel zu dem Materialkorn ausgerichtet ist.

2. System nach Anspruch 1, **dadurch gekennzeichnet, dass** die Öffnung (912) so ausgebildet ist, dass sie sich von dem distalen Ummantelungsabschnitt zu dem Übergangsabschnitt (908) erstreckt.

3. System nach Anspruch 1, **dadurch gekennzeichnet, dass** ein am weitesten distales Ende der medizinischen Vorrichtung (100) proximal zu einem am weitesten distalen Ende des Ummantelungskörperabschnitts angeordnet ist.

4. System nach Anspruch 1, **dadurch gekennzeichnet, dass** die Öffnung (912) etwa 1,5 mm lang ist und nicht mehr als etwa 0,5 mm von dem distalen Ende (902) der Ummantelung (900) entfernt ist.

## Revendications

1. Un système de mise en place (212), comprenant :
un cathéter muni d'une extrémité distale (902) et d'une extrémité proximale (904) ;
une portion de ballon (214) positionnée au niveau de l'extrémité distale du cathéter (12) ;
Un dispositif médical (100), ayant un état comprimé et un état déployé positionné autour de la portion de ballon (214) ; et
une gaine (900) positionnée autour du dispositif médical afin de maintenir le dispositif médical (16, 100) à l'état comprimé,
la gaine (900) comprend :
une portion distale (906) de la gaine située à une extrémité distale (902) de la gaine (900), la portion distale (906) de la gaine ayant un premier diamètre et une première longueur longitudinale ;
une portion de transition (908), d'une seconde longueur longitudinale, ayant une extrémité distale adjacente à l'extrémité proximale de la portion distale de la gaine, l'extrémité distale de la portion de transition (908) ayant un diamètre égal au premier diamètre et ayant une extrémité proximale dont le second diamètre est supérieur au premier diamètre ;
une portion de corps (910) d'une troisième longueur longitudinale, ayant une extrémité distale adjacente à une extrémité proximale de la portion de transition (908), la portion de corps (910) ayant un diamètre égal au second diamètre ;
une ouverture (912) ménagée dans une surface extérieure de la gaine (900), une extrémité la plus distale de l'ouverture (912) étant située à une distance prédéterminée proximalement de l'extrémité distale (902) de la gaine (900) ;
**caractérisé en ce que**
la gaine (900) comprend un matériau ayant un grain orienté selon un axe longitudinal de la gaine, et
où l'ouverture (912) est une fente d'initiation d'une longueur prédéterminée orientée sensiblement parallèle au grain du matériau.

2. Le système selon la revendication 1, **caractérisé en ce que** l'ouverture (912) est prévue de manière à s'étendre de la portion distale de la gaine jusqu'à la portion de transition (908).

3. Le système selon la revendication 1, **caractérisé en ce que** :
une extrémité la plus distale du dispositif médical (100) est proximale à une extrémité la plus distale de la portion de corps de la gaine.

4. Le système selon la revendication 1, **caractérisé en ce que** l'ouverture (912) mesure environ 1,5 mm de long et est positionnée à une distance n'excédant pas environ 0,5 mm de l'extrémité distale (902) de la gaine (900).
